(19) Europäisches Patentamt
European Patent Office
Office européen des brevets

(11) EP 3 415 098 A1

(12) EUROPEAN PATENT APPLICATION
published in accordance with Art. 153(4) EPC

(43) Date of publication:
19.12.2018 Bulletin 2018/51

(51) Int Cl.:
A61B 10/00 (2006.01)

(21) Application number: 17750377.8

(22) Date of filing: 10.02.2017

(86) International application number:
PCT/JP2017/005003

(87) International publication number:
WO 2017/138656 (17.08.2017 Gazette 2017/33)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 10.02.2016 JP 2016024160

(71) Applicant: NeU Corporation
Tokyo 101-0048 (JP)

(72) Inventors:
• FUNANE, Tsukasa
Hiki-gun
Saitama 350-0395 (JP)
• KIGUCHI, Masashi
Hiki-gun
Saitama 350-0395 (JP)
• HASEGAWA, Kiyoshi
Tokyo 105-8717 (JP)

(74) Representative: Strehl Schübel-Hopf & Partner
Maximilianstrasse 54
80538 München (DE)

(54) **LIVING-BODY LIGHT MEASURING DEVICE AND LIVING-BODY LIGHT MEASURING METHOD**

(57) This living-body light measuring device is characterized in including: one or a plurality of light source units each of which irradiate a subject with light modulated using a prescribed modulation frequency; one or a plurality of light detecting units each of which detect an optical signal based on the light radiated from the one or plurality of light sources; a signal acquiring unit which multiplies the optical signal detected by each of the one or plurality of light detecting units by a signal having the prescribed modulation frequency corresponding to the respective light source unit, and which acquires the resulting signal as a signal radiated from the respective light source unit; an analog-digital converter which subjects each signal acquired by the signal acquiring unit to analog-digital conversion using a prescribed sampling frequency; and a computing unit which integrates the signals converted by the analog-digital converter. This living-body light measuring device is also characterized in that the sampling frequency is an even multiple of all the modulation frequencies, and the time over which the computing unit performs the integration is an integral multiple of all the modulated signal periods, being the reciprocal of all the modulation frequencies.

FIG. 5

**Description**

TECHNICAL FIELD

The present invention pertains to a biometric optical measurement apparatus and a biometric optical measurement method.

BACKGROUND ART

Brain function measurement apparatuses using NIRS (Near-Infrared Spectroscopy) are usable as medical and research equipments or usable for checking educational effects/rehabilitation effects, or usable for health management, or usable for a market research of commercial product monitoring. The brain function measurement apparatus is also usable for measuring tissue oxygen saturation and oxygen metabolism in muscles. The brain function measurement apparatus is further usable for a general absorption spectroscope targeting for measuring light scattering substances well as measuring a sugar content of fruit.

The technology described above involves separating and detecting the rays of light from a plurality of light sources, and hence, as disclosed in Patent Document 1, separating and detecting desired signals by irradiating plural rays of light modulated at different frequencies and multiplying a detection signal by a modulation frequency (frequency lock-in detection method). The frequency lock-in detection method in a biometric optical measurement aiming at a multi-point simultaneous measurement has an advantage of making a noise band reducible on one side, but is required to employ light sources having different frequencies sufficiently spaced from each other so as not to cause interferences with each other, i.e., not to cause crosstalk, or is required to dispose the light sources separated spatially at a fixed or larger interval. Patent Document 2 discloses a method (time-division lock-in detection method) of lighting up the light sources at a predetermined modulation frequency. This method has an advantage of not causing the cross talk because the plurality of light sources is not lit up simultaneously.

[Documents of Prior Arts]

[Patent Documents]

[Patent Document 1] Japanese Patent Application Laid-Open Publication No.H9-149903
[Patent Document 2] Japanese Patent Application Laid-Open Publication No. 2008-178563

[Non-Patent Document]

[Non-Patent Document 1] McCormick PW, Stewart M, Lewis G, Dujovny M, Ausman JI, Intracerebral penetration of infrared light, J Neurosurg. 76(2), 315-8 (1992)
[Non-Patent Document 2] Zeff BW, White BR, Dehghani H, Schlaggar BL, Culver JP. Retinotopic mapping of adult human visual cortex with high-density diffuse optical tomography. Proc Natl Acad Sci U S A. 2007;104:12169-12174.

SUMMARY OF THE INVENTION

[Problems to be solved by the Invention]

However, the method disclosed in Patent Document 1 has a problem of upsizing the apparatus as well as setting high a frequency band to generate a large number of frequencies by increasing measurement points. For example, the measurement of a whole head has hitherto entailed using the frequencies of several tens of types . The method of Patent Document 2 has such a problem in principle that light irradiation time is divided and becomes shorter as a number of light sources increases, and therefore a signal-to-noise (S/N) ratio decreases. When downsizing the apparatus adopting the frequency lock-in detection method, the types of frequencies are to be reduced. There is an issue of how the types of frequencies are reduced under a condition of a time-division count being smaller (a best condition is consecutive light) . It is required to select a frequency that does not interfere with a frequency (for example, a 50 Hz band and a 50 kHz band) serving as a noise source, i.e., room illumination and other equivalent noises.

The present invention aims at providing a biometric optical measurement apparatus configured to restrain an S/N ratio and cross talk.

[Means for solving the Problems]

Means given below are adopted for solving the problems.

To be specific, a first aspect is a biometric optical measurement apparatus including:

one or a plurality of light source units to irradiate a measurement examinee's region with rays of light modulated at a predetermined modulation frequency;

one or a plurality of light detection units to detect optical signals based on the rays of light irradiated from the light source units;

a signal acquiring unit to multiply the optical signal detected by the light detection unit by a signal of the predetermined modulation frequency corresponding to each light source unit, and to acquire the multiplied signal as a signal irradiated from each light source unit;

an analog-to-digital (AD) converter to AD-convert the signal acquired by the signal acquiring unit at a predetermined sample frequency; and

an arithmetic unit to integrate the signals converted by the AD converter,

the sample frequency being an even-numbered multiple of all the modulation frequencies,

time integrated by the arithmetic unit being an integral multiple of a modulation signal cycle as an inverse number of the modulation frequency.

The aspect of the disclosure may be attained by an information processing apparatus that runs a program. In other words, a configuration of the disclosure may be specified as a program for making the information processing apparatus execute processes to be executed respective means in the aspect described above, or as a non-transitory computer readable recording medium recorded with the program. The configuration of the disclosure may also be specified as a method by which the information processing apparatus executes the processes to be executed by the respective means . The configuration of the disclosure may further be specified as a system including the information processing apparatus that executes the processes to be executed by the respective means.

Steps describing the program include, of course, processes to be executed in time series along a written sequence, and, even when not necessarily processed in time series, the processes to be executed in parallel or individually. A part of the steps describing the program may be omitted.

[Effect of the Invention]

According to the present invention, it is feasible to provide the biometric optical measurement apparatus configured to restrain the S/N ratio and the cross talk.

BRIEF DESCRIPTION OF THE DRAWINGS

FIG. 1 is a diagram illustrating a configuration involved in information processing of an information processing system according to one embodiment of the present invention.

FIG. 2 is a diagram illustrating an example of a configuration of a head region attaching device.

FIG. 3 is a diagram illustrating an example of a configuration of a user terminal.

FIG. 4 is a diagram illustrating an Example 1 of a light source module and a detection module.

FIG. 5 is a diagram illustrating an Example 2 of the light source module and the detection module.

FIG. 6 is a diagram illustrating a section of a head region of a human body attached with the head region attaching device.

FIG. 7 is a graphic chart illustrating an example of timings of irradiating the rays of light from the light source module.

FIG. 8 is a diagram illustrating an Example 1 of a layout of the light source modules and the detection modules.

FIG. 9 is a diagram illustrating an Example 2 of the layout of the light source modules and the detection modules.

FIG. 10 is a table illustrating an Example 1 of a relationship between the module layout, a minimum time division count and a minimum modulation frequency count.

FIG. 11 is a table illustrating an Example 2 of a relationship between the module layout, the minimum time division count and the minimum modulation frequency count.

FIG. 12 is a diagram illustrating an example, in which the modules are disposed in a parallelogram shape at lattice points of a triangular lattice.

FIG. 13 is a table illustrating an example of a relationship between the module layout of the triangular lattice, the minimum time-division count and the minimum modulation frequency count.

FIG. 14 is a diagram illustrating an example of a layout, corresponding to a multi-distance measurement, of the light source modules and the detection modules.

FIG. 15 is a flowchart illustrating an example of an operation flow of setting the frequency in the module.

## DESCRIPTION OF THE EMBODIMENTS

An embodiment will hereinafter be described with reference to the drawings. A configuration of the embodiment is an exemplification, and a configuration of the present invention is not limited to the specific configuration of the embodiment. On the occasion of carrying out the invention, specific configurations may be adopted corresponding to the embodiments.

[Embodiment]

(Example of Configuration)

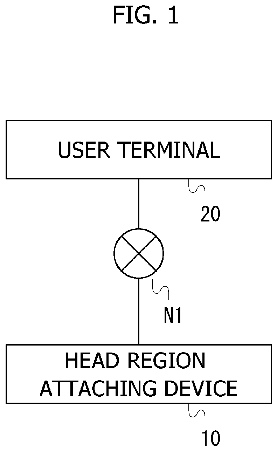

FIG. 1 is a diagram illustrating a configuration involved in information processing of an information processing system according to one embodiment of the present invention. As in FIG. 1, the information processing system includes a head region attaching device 10 and a user terminal 20. The information processing system detects measurement data representing variations in bloodflow rate and/or variations in haemodynamics from a head region of a user, and acquires brain activity information representing brain activity states of the user. The head region attaching device 10 and the user terminal 20 may be configured to perform communications via a network NI, and may also be configured integrally. The information processing system is one example of a "biometric optical measurement apparatus".

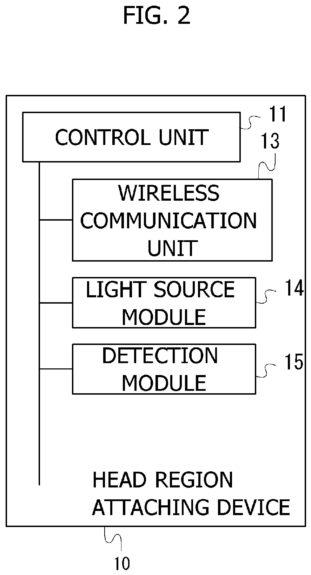

FIG. 2 is a diagram illustrating an example of a configuration of the head region attaching device. The head region attaching device 10 includes, an an aspect of the information processing, a control unit 11, a wireless communication unit 13, a light source module 14, and a detection module 15. The control unit 11 controls measurements by and communications with the head region attaching device 10. The control unit 11 includes; e.g., a processor instanced by a CPU (Central Processing unit) or a DSP (Digital Signal Processor); and a memory, and executes processes based on a computer program, firmware and other equivalent software components deployed in an executable manner on the memory. However, the control unit 11 may also be a dedicated hardware circuit, an FPGA (Field Programmable Gate Array) and other equivalent elements that execute a cooperative process with each component by actuating the wireless communication unit 13, the light source module 14 and the detection module 15. The control unit 11 may also be configured as a mixture of the CPU, the DSP, the dedicated hardware circuit and other equivalent elements. The head region attaching device 10 may have a storage means to store data and other equivalent information used in the head region attaching device 10.

The head region attaching device 10 has a structure of being attached to a head region of a user by being wound on the head region in a headband-like shape, and fixed to the head region of the user by fastening a fixing member.

The wireless communication unit 13 is connected via a predetermined interface to the control unit 11, the light source module 14 and the detection module 15. However, the wireless communication unit 13 may be configured to acquire the data from the detection module 15 via the control unit 11. The wireless communication unit 13 performs the communications with the user terminal 20 via the network N1. The network N1 is a network pursuant to standards instanced by Bluetooth (registered trademark), wireless LAN (Local Area Network) and ZigBee. The wireless communication unit 13 is one example of "transfer means". It does not, however, mean that there is a limit to the standards of the wireless interface of the wireless communication unit 13 in the information processing system.

When performing the communications via the network N1, an identifier for identifying the head region attaching device 10 is embedded into a header field of a communication header, or a user data field (payload field) of communication data, thereby enabling the user terminal 20 to identify the user (measurement examinee).

In the information processing system, the wireless communication unit 13 may be provided with a communication unit to perform wired communications in place of the wireless communication unit 13 or together with the wireless communication unit 13. In other words, the head region attaching device 10 may be connected to the user terminal 20 via an interface for the wired communications. It does not mean that there is a limit to the interface for the wired communications in this case; and a variety of interfaces instanced by USB (Universal Serial Bus) and PCI Express are, however, usable corresponding to applications of the information processing system.

The light source module 14 modulates visible rays of light or near infrared-rays of light at a predetermined frequency, and irradiates the head region with these rays of light at predetermined timing. A light source of the visible rays of light or the near infrared-rays of light is exemplified by LED (Light Emitting Diodes), a visible ray lamp and an infrared-ray lamp. Each light source is identified by, e.g., identifier. The head region attaching device 10 may include a plurality of light source modules 14.

The detection module 15 receives the visible rays of light or the near infrared-rays of light irradiated over the head region, partially absorbed and scattered in the vicinity of a cerebral cortex of a brain, and converts the scattered rays of light into electric signals. The cerebral cortex of the brain has different variations in bloodflow rate and/or variations in haemodynamics, e.g., depending on the brain activity states. As a result, there vary a quantity of haemoglobin bound to oxygen in the blood and a quantity of haemoglobin not bounded to oxygen in respective regions of the cerebral cortex. An absorptive characteristic or a scattering characteristic of the visible rays of light or the near infrared-rays of light in the vicinity of the cerebral cortex of the brain, varies due to variations in quantity of haemoglobin, variations in quantity of oxygen and other equivalent variations. The detection module 15 converts such visible rays of light or near infrared-rays of light into the electric signals that light quantities vary depending on variations in absorptivity or transmittance of the visible rays of light or the near infrared-rays of light in accordance with a state of the bloodflow and/or a state of haemodynamics in the vicinity of the cerebral cortex, and outputs the converted electric signals.

The detection module 15 includes a light source for the visible rays of light that irradiates the visible rays of light or a light source for the near infrared-rays of light that irradiates the near infrared-rays of light, and a detector to receive the visible rays of light or the near infrared-rays of light. A light receiving unit includes: a photo-electric element instanced by a photo diode, an avalanche photo diode, a photomultiplier tube and a photo transistor; an amplifier; and an AD (Analog-to-Digital) converter.

The light source module 14 for the visible rays of light or the near infrared-rays of light and the detection module 15 may be provided not to be paired. For example, a plurality of detection modules 15 may be provided for one light source module 14 for the visible rays of light or the near infrared-rays of light. The light source module 14 is an example of 'light irradiation means". The detection module 15 is one example of "light detection means".

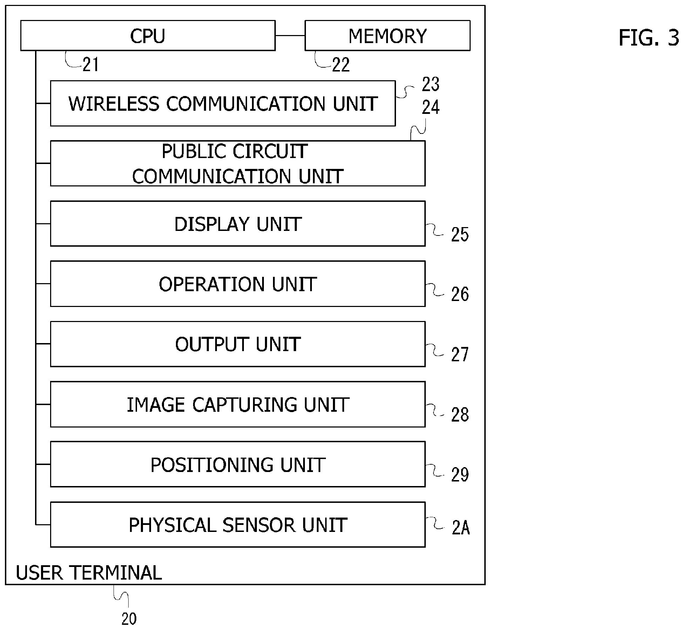

FIG. 3 is a diagram illustrating an example of a configuration of the user terminal. The user terminal 20 acquires variation data of the absorptivity or transmittance of the visible rays of light or the near infrared-rays of light in the vicinity of the cerebral cortex of the user from the head region attaching device 10, and provides a service encompassing a variety of information processes pertaining to the brain activity states of the user. The user terminal 20 is one example of an "information processing apparatus (computer)". The user terminal 20 is attainable by using a dedicated or general-purpose computer instanced by a PC (Personal Computer), a smartphone, a mobile phone, a tablet terminal, a car navigation system, a PDA (Personal Digital Assistant) and a game machine (amusement device), or an electronic equipment mounted with the computer. The user terminal 20 is installable in, e.g., a fitness club, a private cram school, and other equivalent facilities.

The user terminal 20 includes a CPU 21, a memory 22, a wireless communication unit 23, a public circuit communication unit 24, a display unit 25, an operation unit 26, an output unit 27, an image capturing unit 28, a positioning unit 29, and a physical sensor unit 2A. The CPU 21 executes processes as the user terminal 20, based on a computer program deployed in the executable manner on the memory 22. The processes as the user terminal 20 are defined as, e.g., the service encompassing the variety of information processes pertaining to the brain activity states of the user. The CPU 21, which runs such a computer program, is one example of "analyzing means".

The memory 22 stores the computer program to be run by the CPU 21 or the data to be processed by the CPU 21. The memory 22 may include a volatile memory and a nonvolatile memory. The memory 22 is one example of "storage means".

The wireless communication unit 23 is the same as the wireless communication unit 13 of the head region attaching device 10. The wireless communication unit 23 is one example of "receiving means". The user terminal 20 may include a communication unit to perform wired communications in place of the wireless communication unit 23 or together with the wireless communication unit 23.

The public circuit communication unit 24 performs the communications with a server (information processing apparatus) and other equivalent apparatuses on a host network via this host network (unillustrated). The host network is a public circuit network, e.g., a mobile phone network. The host network is the mobile phone network, in which case the public circuit communication unit 24 establishes a connection to the host network via a base station of the mobile phone network. However, the host network may also be a network including: an access network to a communication apparatus of an Internet provider; and the Internet. The access network to the communication apparatus of the Internet provider is instanced by an optical network and an ADSL (Asymmetric Digital Subscriber Line) provided by a common carrier. The host network is one example of a "public wireless network". The public circuit communication unit 24 is one example of "public wireless communication means". It does not, however, mean that the host network is limited to the public circuit network in the information processing system; and the host network may also be an in-house network instanced by a LAN (Local Area Network), a private circuit of an enterprise, a business operator, a government office, a school, a research institute and other equivalent facilities, and a wide area network (WAN) instanced by a VPN (Virtual Private Network) . The enterprise, the business operator, the government office, the school, the research institute and other equivalent facilities will hereinafter be simply termed the enterprise and other equivalent facilities.

The display unit 25 is instanced by a liquid crystal display and an EL (Electro-Luminescence) panel, and displays information outputted from the CPU 21. The operation unit 26 is instanced by a push button and a touch panel, and accepts user's operation. The output unit 27 is instanced by a vibrator to output vibrations and a loud speaker to output sounds or voices . The image capturing unit 28 is a camera including, a solid-state image capturing element. A CCD (Charge Coupled Device) image sensor, a CMOS (Complementary Metal Oxide Semiconductor) image sensor and other equivalent sensors are available as the solid-state image capturing element.

The positioning unit 29, which is, e.g., a GPS (Global Positioning System) receiver, calculates a present position (latitude, longitude, and other equivalent geographical coordinates) and the time by receiving radio waves from a GPS satellite. It does not, however, mean that the positioning unit 29 is limited to a unit having the GPS receiver. For example, the public circuit communication unit 24 is the mobile phone network, in which case the positioning unit 29 may execute positioning based on a distance from the mobile phone base station.

The physical sensor unit 2A is, e.g., an acceleration sensor or an angular acceleration sensor or other equivalent sensors. However, the physical sensor unit 2Amay also be a temperature sensor, a humidity sensor, an atmospheric pressure sensor, or a water pressure sensor.

<Example 1 of Light Source Module and Detection Module>

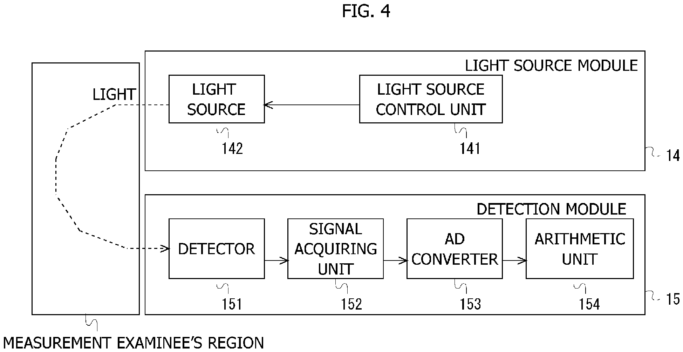

FIG. 4 is a diagram illustrating an Example 1 of the light source module and the detection module. The light source module 14 in FIG. 4 includes a light source control unit 141 and a light source 142. The detection module 15 in FIG. 4 includes a detector 151, a signal acquiring unit 152, an AD converter 153, and an arithmetic unit 154.

In the light source module 14, the light source control unit 141 controls the rays of light (visible rays of light or near infrared-rays of light) irradiated from the light source 142. The light source 142 irradiates a measurement examinee's region instanced by the head region of a human body with the rays of light, of which intensity is modulated at a predetermined frequency.

In the detection module 15, the detector 151 detects the rays of light propagating through the measurement examinee's region. The signal acquiring unit 152 acquires the detected rays of light as signals. The AD converter 153 converts the signals acquired by the signal acquiring unit 152 into digital data. The arithmetic unit 154 executes a process of adding or averaging the digital data converted by the AD converter 153.

<Example 2 of Light Source Module and Detection Module>

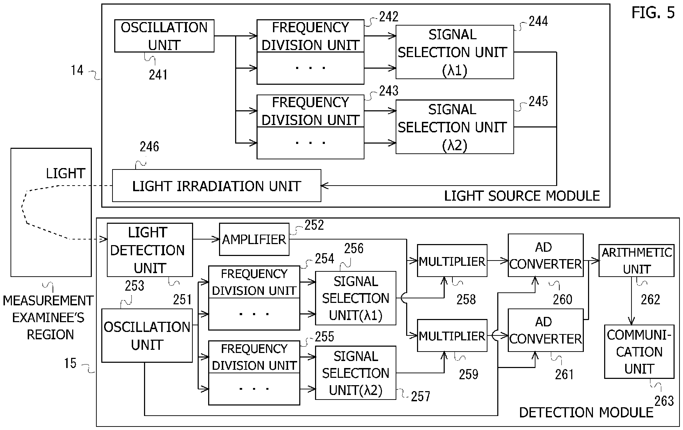

FIG. 5 is a diagram illustrating an Example 2 of the light source module and the detection module. The light source module 14 in FIG. 5 includes an oscillation unit 241, a frequency division unit 242, a frequency division unit 243, a signal selection unit 244, a signal selection unit 245, and a light irradiation unit 246. The detection module 15 in FIG. 5 includes a light detection unit 251, an amplifier 252, an oscillation unit 253, a frequency division unit 254, a frequency division unit 255, a signal selection unit 256, a signal selection unit 257, a multiplier 258, a multiplier 259, an AD converter 260, an AD converter 261, an arithmetic unit 262, and a communication unit 263. The multiplier is also called a mixer (MIXER) . The multiplier is one example of a "signal acquiring unit".

In the light source module 14, the oscillator 241 generates clock signals having a predetermined frequency. Each of the frequency division units 242, 243 generates a plurality of clock signals by dividing a frequency of the clock signal generated by the oscillator 241. The signal selection unit 244 selects the clock signals being frequency-divided by the frequency division unit 242, corresponding to a timing of predetermined time, thereby generating the clock signals having a predetermined frequency for modulating rays of light having a wavelength $\lambda 1$, which are irradiated by the light irradiation unit 246. The frequency of the signal selected by the signal selection unit 244 is different from the frequency of the signal selected by the signal selection unit 245. The light irradiation unit 246 generates rays of light having the wavelength $\lambda 1$ and rays of light having a wavelength $\lambda 2$, and modulates the generated rays of light at the frequencies of the signals selected by the signal selection units 244, 245, thereby irradiating the modulated rays of light over the measurement examinee's region. The rays of light having the plurality of wavelengths are irradiated from one light source, whereby plural types of results with respect to one position of the measurement examinee's region are obtainable . The rays of light having the two types of wavelengths are herein used, and the wavelengths are not, however, limited to the two types . The light irradiation unit 246 irradiates the rays of light at predetermined time of preset time-division.

In the detection module 15, the light detection unit 251 detects the rays of light propagating through the measurement examinee's region. The amplifier 252 amplifies the rays of light detected by the light detection unit 251. The oscillation unit 253 generates the clock signals having the same predetermined frequency as the frequency of the oscillation unit 241. Each of the frequency division units 254, 255 generates the plurality of clock signals by dividing the frequency of the clock signal generated by the oscillator 253. The signal selection unit 256 selects the clock signals being frequency-divided by the frequency division unit 254, corresponding to the timing of predetermined time, thereby generating the clock signals having the predetermined frequency at which to modulate the rays of light having the wavelength $\lambda 1$, which are detected by the light detection unit 251. The signal selection unit 257 selects the clock signals being frequency-divided by the frequency division unit 255, corresponding to the timing of predetermined time, thereby generating the clock signals having the predetermined frequency at which to modulate the rays of light having the wavelength λ2, which are detected by the light detection unit 251. The multiplier 258 multiplies the clock signal (clock reference signal) generated by the signal selection unit 256 by the signal amplified by the amplifier 252. The multiplier 258 smooths (alternatively, an analog lowpass filter is applied) the multiplied signal. The multiplier 259 multiplies the clock signal (clock reference signal) generated by the signal selection unit 257 by the signal amplified by the amplifier 252. The multiplier 259 smooths (alternatively, the analog lowpass filter is applied) the multiplied signal. The AD converter 260 converts the signal processed by the multiplier 258 into a digital signal. The AD converter 261 converts the signal processed by the multiplier 259 into the digital signal. The arithmetic unit 262 executes predetermined signal processing for the converted digital signal. The communication unit 263 outputs the signal processed by the arithmetic unit 262. The oscillation units 241, 253 may be commonized. The oscillation units are commonized, thereby enabling a number of components to be reduced while minimizing the clock signals to be generated, and enabling the measurement with a crosstalk being reduced to be attained. The oscillation unit may also exist for every group of clocks signals generated by each signal signal selection unit.

<Distance between Light Source and Detector>

It is preferable that a distance between the light source (light irradiation unit) of the light source module 14 and the detector (light detection unit) of the detection module 15 is set to about 30 mm. It is feasible to detect the signal derived from the cerebral cortex of the head region of the human body from above a skin by setting the distance to about 30 mm (refer to Non-Patent Document 1) .

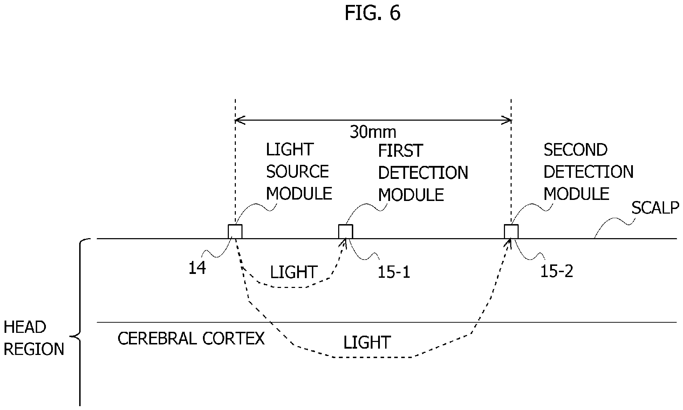

FIG. 6 is a diagram illustrating a section of the head region of the human body attached with the head region attaching device. Depicted in FIG. 6 are the head region of the human body, the light source module 14, a first detection module 15-1 and a second detection module 15-2. A distance between the light source module 14 and the second detection module 15-2 is set to 30 mm, and a distance between the light source module 14 and the first detection module 15-1 is well shorter (e.g., less than 10 mm) than 30 mm. The light source module 14 irradiates the rays of light over the head region. The rays of light irradiated from the light source module 14 propagate through the head region, and are detected by the first detection module 15-1 and the second detection module 15-2. The rays of light detected by the second detection module 15-2 reach the cerebral cortex distanced from a scalp. While on the other hand, the rays of light detected by the first detection module 15-1 do not reach the cerebral cortex. In other words, even when the first detection module 15-1 detects the rays of light irradiated from the light source module 14, the rays of light do not become the signals of the cerebral cortex. Hence, the distance between the light source module 14 and the detection module 15 is set to about 30 mm, thereby enabling the signals derived from the cerebral cortex to be detected. Note that the distance between the light source module 14 and the first detection module 15-1 may be set to about 15 mm - 21 mm, in which case also a contribution of the signals derived from the cerebral cortex to the signals detected by the first detection module 15-1 and the second detection module 15-2 is different from a contribution of the signals derived from the scalp thereto, and it is therefore feasible to presume the signals derived from the cerebral cortex by using two types of signals.

When the distance between the light source module 14 and the detection module 15, which are attached to the head region of the human body, is extended to 50 mm from 30 mm, an intensity of the rays of light to be detected becomes approximately one hundredth (refer to Non-Patent Document 2). In other words, the distance is considered to become approximately one tenth each time when distanced by 10 mm. Supposing herein that the rays of light irradiated from the light source module 14, travelling through the head region and detected by the detection module 15 vary by about several percent (%) corresponding to the variations in bloodflow rate of the brain, the detection module 15 detecting the rays of light from the light source module 14 distanced by 30 mm receives the rays of light from the light source module 14 distanced by 65 mm or longer from the detection module 15, of which the intensity becomes equal to or smaller than one thousandth (equal to or smaller than 0.1%) of the intensity of the rays of light from the light source module 14 distanced by 65 mm or longer from the detection module 15 and is therefore ignorable in fact. Accordingly, it is preferable that the plurality of light source modules 14 irradiating the rays of light, modulated at the same frequency, at the same timing does not exist in a range demarcated by a radius of smaller than 65 mm with the detection module 15 being centered.

In a case that the light source module 14 is not tightly fitted to the head region defined as the measurement examinee's region but floats and in other equivalent cases, it might happen that the rays of light from the light source module 14 propagates not through the head region but through air and are detected by the detection module 15. When taking this phenomenon into consideration, it is preferable that the plurality of light source modules 14 irradiating the same rays of light at the same timing does not exist in the range demarcated by the radius of smaller than 150 mm with the detection module 15 being centered.

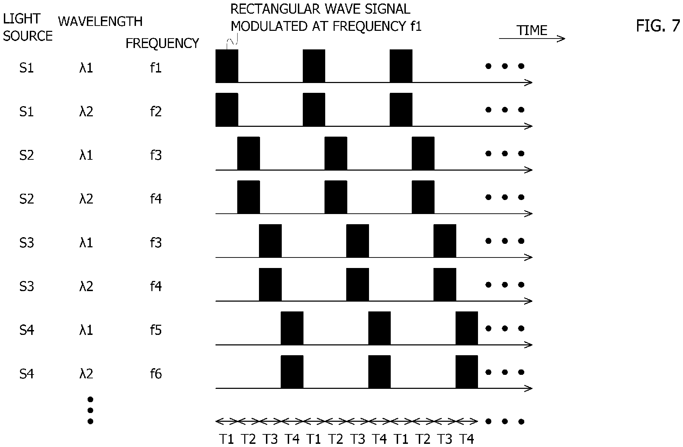

FIG. 7 is a graphic chart illustrating an example of the timings of irradiating the rays of light from the light source module . In graphs of FIG. 7, an axis of abscissa is a timing axis, along which a solid black square on the timing-axis indicates a time at which the rays of light are irradiated. In FIG. 7, symbols S1 - S4 represents the light source modules irradiating the rays of light. The graphs of FIG. 7 are such that, e.g., in a light source S1, the rays of light having the wavelength $\lambda1$ are modulated at a frequency f1, the rays of light having the wavelength A2 are modulated at a frequency f2, and these rays of light are irradiated at a timing of a time T1. In a light source S2, the rays of light having the wavelength $\lambda1$ are modulated at a frequency f3, the rays of light having the wavelength A2 are modulated at a frequency f4, and these rays of light are irradiated at a timing of a time T2. In a light source S3, the rays of light having the wavelength $\lambda1$ are modulated at the frequency f3, the rays of light having the wavelength A2 are modulated at the frequency f4, and these rays of light are irradiated at a timing of a time T3. In a light source S4, the rays of light having the wavelength $\lambda1$ are modulated at a frequency f5, the rays of light having the wavelength $\lambda2$ are modulated at a frequency f6, and these rays of light are irradiated at a timing of a time T4. The example in FIG. 7 is that a time-division count is "4", and the respective times of the time-division are consecutive in a sequence of the time T1, the time T2, the time T3 and the time T4. The time T4 is followed by the time T1. In one light source, the rays of light having the wavelength $\lambda1$ and the rays of light having the wavelength A2 are modulated at the frequencies different from each other . The light irradiation time and the light modulation frequency are determined so that the rays of light modulated at the same frequency are not irradiated at the same timing in the predetermined range from the detection module.

<Layout of Modules>

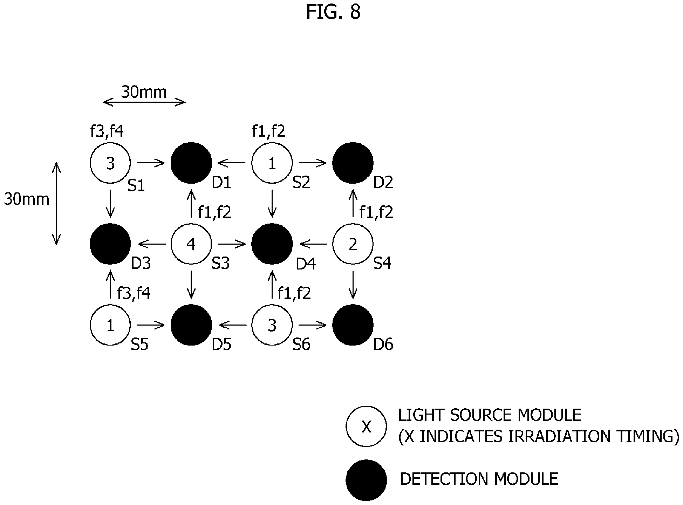

FIG. 8 is a diagram illustrating an Example 1 of a layout of the light source modules and the detection modules. The module layout in FIG. 8 is a module layout as viewed from outside when the head region attaching device is attached to the measurement examinee's region. The example in FIG. 8 is that totally 12 modules are laid out, in which specifically the modules are aligned by fours in three rows in a crosswise direction of a tetragonal lattice, i.e., the modules are aligned by threes in four columns in a vertical direction of the tetragonal lattice. In the example of FIG. 8, the light source module 14 is disposed at a left upper edge, and the light source modules 14 and the detection modules 15 are alternately disposed in the crosswise direction. The light source modules 14 and the detection modules 15 are also alternately disposed in the vertical direction. Therefore, the module layout in FIG. 8 encompasses the six light source modules 14 (S1 - S6) and the six detection modules 15 (D1 - D6). Numerals written inside of the light source modules in FIG. 8 indicate the timings (time-division times) at which the rays of light are irradiated. Supposing that a division count of the time-division is "4", the irradiation timings are given in the sequence of 1, 2, 3, 4 followed in loop by 1, and this sequence is repeated. A first period of time is notated by a time T1, and a second period of time is likewise notated by a time T2. Symbols [f1, f2] written in the periphery of the light source module indicate the frequencies at which the wavelengths $\lambda1$, A2 (of the rays of light) to be irradiated are modulated. For example, in the light source module 14 of S1, the rays of light having the wavelength $\lambda1$ are modulated at the frequency f3 and then irradiated at a third period of time (T3); and the rays of light having the wavelength A2 are modulated at the frequency f4 and then irradiated at T3. The rays of light irradiated from the light source module 14 travel through the measurement examinee's region and are detected by the neighboring detection module 15. For instance, the rays of light irradiated by the light source module 14 of S1 are detected by the light source modules 14 of D1, D3 and then processed. In one light source module, the rays of light having the wavelengths $\lambda1$, A2 are modulated at the frequencies different from each other, and are irradiated at the same time (timing).

Each detection module 15 detects signals of the rays of light irradiated by the light source module 14 and travelling through the measurement examinee's region. For example, the detection module 15 of D4 detects the rays of light having the wavelengths $\lambda1$, A2 that are irradiated from the light source module 14 of S2 at the time T1 and modulated at the frequencies f1, f2, detects the rays of light having the wavelengths $\lambda1$, $\lambda2$ that are irradiated from the light source module 14 of S4 at the time T2 and modulated at the frequencies f1, f2, detects the rays of light having the wavelengths $\lambda1$, $\lambda2$ that are irradiated from the light source module 14 of S6 at the time T3 and modulated at the frequencies f1, f2, and detects the rays of light having the wavelengths $\lambda1$, $\lambda2$ that are irradiated from the light source module 14 of S3 at the time T4 and modulated at the frequencies f1, f2. The detection module 15 of D4, though capable of detecting the rays of light from the light source module 14 of S1 at, e.g., the time T3 together with the rays of light from the light source module 14 of S6, is enabled to detect only the rays of light from the light source module 14 of S1 because the modulation frequency of the rays of light from the light source module 14 of S6 is different from the modulation frequency of the rays of light from the light source module 14 of S1. To be specific, the rays of light detected for the time T3 are multiplied by the modulation frequency of the light source module 14 of S6, thereby enabling elimination of the signals related to other modulation frequencies. Hence, even when the light source module 14 to perform the irradiation at the same timing exists in the vicinity of the detection module 15 and when the modulation frequency is different, it is feasible to separate and extract the rays of light.

The detection module 15 of D1 detects the rays of light having the wavelengths $\lambda1$, $\lambda2$ that are irradiated from the light source module 14 of S2 at the time T1 and are modulated at the frequencies f1, f2, detects the rays of light having the wavelengths λ1, λ2 that are irradiated from the light source module 14 of S1 at the time T3 and are modulated at the frequencies f3, f4, and detects the rays of light having the wavelengths λ1, λ2 that are irradiated from the light source module 14 of S3 at the time T4 and are modulated at the frequencies f1, f2. Herein, the detection module 15 of D1 performs averaging by multiplying the rays of light detected at the time T1 by the clock signal having the frequency f1, and extracts the rays of light having the wavelength λ1 from the light source module 14 of S2. The detection module 15 of D1 performs averaging by multiplying the rays of light detected at the time T1 by the clock signal having the frequency f2, and extracts the rays of light having the wavelength λ2 from the light source module 14 of S2. Similarly, the rays of light from the light source module 14 of S1 are extracted out of the rays of light detected at the time T3, and the rays of light from the light source module 14 of S3 are extracted out of the rays of light detected at the time T4. Hereat, a sampling frequency is set to a predetermined frequency that will be described later, thereby enabling the elimination of the signals related to other modulation frequencies more efficiently.

The rays of light having the two wavelengths are herein used, and the wavelengths may, however, take three or more types without being limited to the two types . In this case, three or more modulation frequencies are used for one light source module 14. One type of wavelength may also be available.

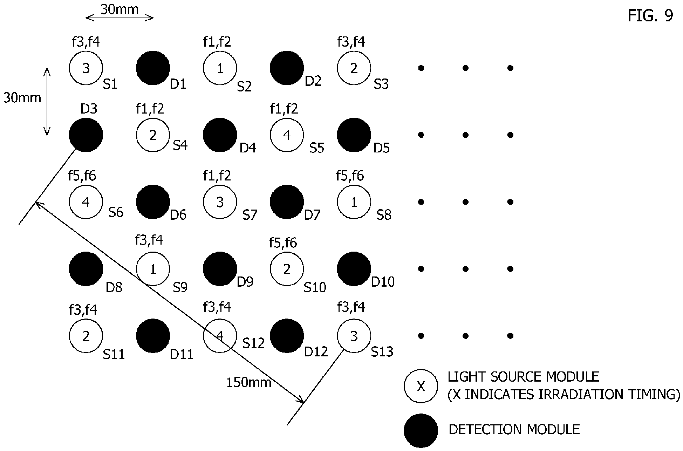

FIG. 9 is a diagram illustrating an Example 2 of the layout of the light source modules and the detection modules. The module layout in FIG. 9 is a module layout as viewed from outside when the head region attaching device is attached to the measurement examinee's region. The example in FIG. 9 is that the modules are aligned by fives or more in five rows in the crosswise direction of the tetragonal lattice, i.e., the modules are aligned by fives in five or more columns in the vertical direction of the tetragonal lattice. In the example of FIG. 9, the light source module 14 is disposed at the left upper edge, and the light source modules 14 and the detection modules 15 are alternately disposed in the crosswise direction. The light source modules 14 and the detection modules 15 are also alternately disposed in the vertical direction. The numerals written inside of the light source modules and the symbols instanced by [f1, f2] written in the peripheries of the light source modules in FIG. 9 are the same as those in the example of FIG. 8. An interval between the neighboring modules is 30 mm. The light source irradiating the rays of light modulated at the same modulation frequency at the same irradiation timing with respect to each detection module is not disposed at a distance of less than 150 mm from each detection module. With this contrivance, e.g., the detection module 15 of D3 is enabled to detect the rays of light modulated at the modulation frequencies f3, f4 from the light source module 14 of S1 and the light source module 14 of S13 at the timing of the time T3, and the intensity of the rays of light from the light source module 14 of S13 is far smaller than the intensity of the rays of light from the light source module 14 of S1 because the light source module 14 of S13 is distanced by 150 mm or longer. Therefore, the detection module 15 of D3 is hardly affected by the rays of light from the light source module 14 of S13. The layout of FIG. 9 disables the rays of light modulated at the same frequency from being irradiated at the same timing in the range of being less than 150 mm from the detection module on condition that the time division count is "4", and the modulation frequencies take three types with respect to one wavelength (herein, six types because of the two wavelengths). In other words, this configuration does not entail preparing the modulation frequencies for the number of the light source modules, and enables the modules to be down-sized and circuits for generating the frequencies to be reduced.

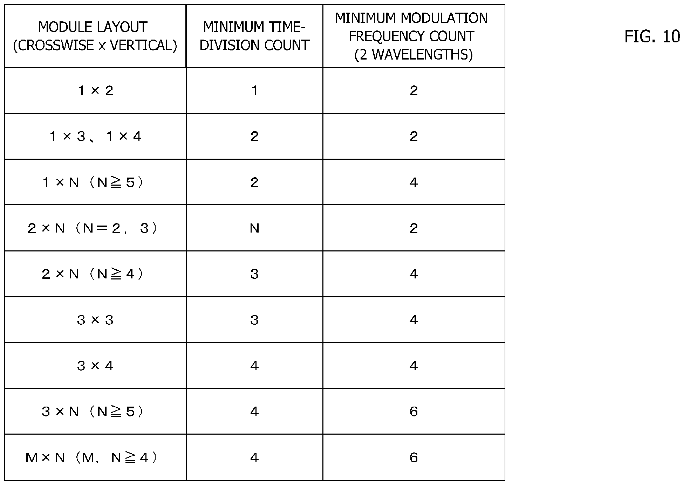

FIG. 10 is a table illustrating an Example 1 of a relationship between the module layout, a minimum time division count and a minimum modulation frequency count. In FIG. 10, the module layout is such a layout that the modules are disposed at lattice points of the tetragonal lattice as in the examples of FIGS. 8 and 9. The interval of the tetragonal lattice is 30 mm. In each module layout, as in the examples of FIGS. 8 and 9, the light source module is disposed at the left upper edge, and the light source modules and the detection modules are alternately disposed both in the crosswise direction and in the vertical direction. The example in FIG. 10 is an example given when the two or more light source modules having the same timing (the same time of time-division) and the same modulation frequency are not disposed in (a range demarcated by) a radius of less than 150 mm from the detection module . For example, when a probe layout is given by "1 x 4" (i.e., the two light source modules and the two detection modules are alternately disposed in one column), the minimum time division count is "2", and the minimum modulation frequency count is "2". In the example of FIG. 10, the rays of light used in the light source module 14 have the two types of wavelengths. Accordingly, in the "1x 4" layout, the two modulation frequencies are used for the rays of light having the two wavelengths by being time-divided into two, thereby enabling the rays of light modulated at the same frequency not to be irradiated at the same timing in the range of less then 150 mm from the detection module. A priority is given to minimizing the time-division count in the example of FIG. 10. This is because an S/N (Signal-to-Noise) ratio decreases as the time-division count increases. In an "$M \times N(M,N \geq 4)$" layout, the six modulation frequencies are used for the rays of light having the two wavelengths by being time-divided into four, thereby enabling the rays of light modulated at the same frequency not to be irradiated at the same timing in the range of less then 150 mm from the detection module. In other words, the time-division count "4" and the modulation frequency count "6" enable measurements to be done in any layouts of the tetragonal lattice. The relationship between the module layout, the minimum time division count and the minimum modulation frequency count is stored in the storage means and other equivalent means of the head region attaching device 10 and the user terminal 20.

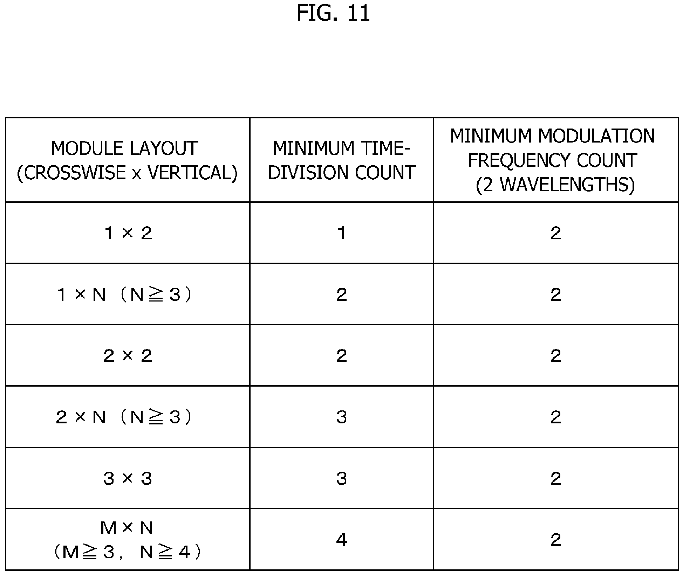

FIG. 11 is a table illustrating an Example 2 of the relationship between the module layout, the minimum time-

division count and the minimum modulation frequency count. In FIG. 11, the module layout is such a layout that the modules are disposed at the lattice points of the tetragonal lattice as in the examples of FIGS. 8 and 9. The interval of the tetragonal lattice is 30 mm. In each module layout, as in the examples of FIGS. 8 and 9, the light source module is disposed at the left upper edge, and the light source modules and the detection modules are alternately disposed both in the crosswise direction and in the vertical direction. The example in FIG. 11 is an example given when the two or more light source modules having the same timing (the same time of time-division) and the same modulation frequency are not disposed in a range demarcated by the radius of less than 65 mm from the detection module. For example, when the probe layout is given by "2xN (N≥4)" (i.e., an N-number of light source modules and an N-number of detection modules are alternately disposed in two columns), the minimum time-division count is "3", and the minimum modulation frequency count is "2". In the example of FIG. 11, the rays of light used in the light source module 14 have the two types of wavelengths. Hence, in the "2 x N" layout, the two modulation frequencies are used for the rays of light having the two wavelengths by being time-divided into three, thereby enabling the rays of light modulated at the same frequency not to be irradiated at the same timing in the range of less then 65 mm from the detection module. The priority is given to minimizing the time-division count in the example of FIG. 11. In an "M×N(M≥3, N≥4)" layout, the two modulation frequencies are used for the rays of light having the two wavelengths by being time-divided into four, thereby enabling the rays of light modulated at the same frequency not to be irradiated at the same timing in the range of less then 65 mm from the detection module. In other words, the time-division count "4" and the modulation frequency count "2" enable the measurements to be done in any layouts of the tetragonal lattice.

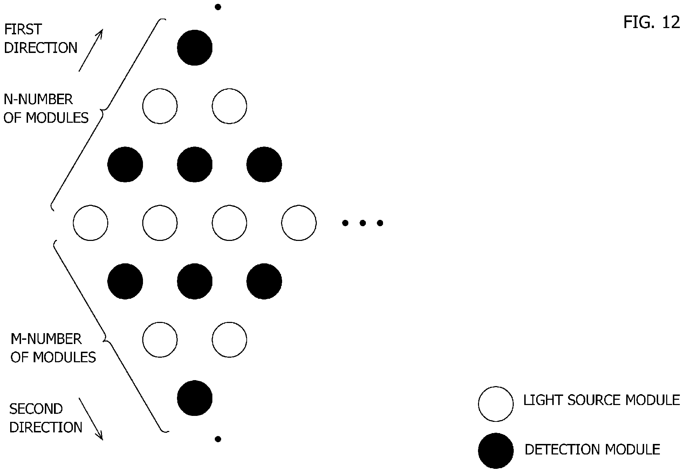

FIG. 12 is a diagram illustrating an example, in which the modules are disposed in a parallelogram shape at the lattice points of a triangular lattice. The module layout in FIG. 12 is a module layout as viewed from outside when the head region attaching device is attached to the measurement examinee's region. In the example of FIG. 12, an N-number of modules are disposed in a first direction of the triangular lattice in an M-number of rows arranged in a second direction angled at 120 degrees to the first direction. In the example of FIG. 12, the light source module 14 is disposed at the left edge, and the light source modules 14 and the detection modules 15 are alternately disposed both in the first direction and in the second direction. The interval between the neighboring modules is 30 mm.

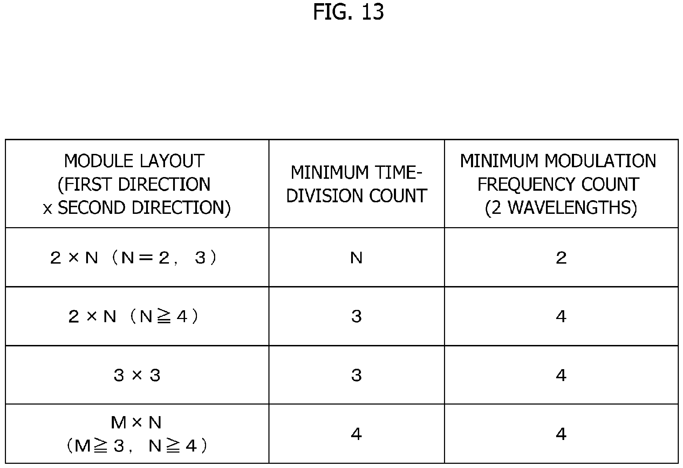

FIG. 13 is a table illustrating an example of a relationship between the module layout of the triangular lattice, the minimum time-division count and the minimum modulation frequency count. In FIG. 13, the module layout is such a layout that the respective modules are, as in the example of FIG. 12, disposed in the parallelogram shape at the lattice points of the triangular lattice. An interval of the triangular lattice is 30 mm. In each module layout, as in the example of FIG. 12, the light source module is disposed at the left edge, and the light source modules and the detection modules are alternately disposed both in the first direction and in the second direction. The example of FIG. 13 is an example of preventing the two or more light source modules having the same timing (the same time of time-division) and the same modulation frequency from being disposed in (the range demarcated by) the radius of less than 65 mm from the detection module. In a "first direction M x second direction N (M≥3, N≥4)" layout, the four modulation frequencies are used for the rays of light having the two wavelengths by being time-divided into four, thereby enabling the rays of light modulated at the same frequency not to be irradiated at the same timing in the range of less then 65 mm from the detection module. In other words, the time-division count "4" and the modulation frequency count "4" enable the measurements to be done in any layouts of the triangular lattice taking the parallelogram shape.

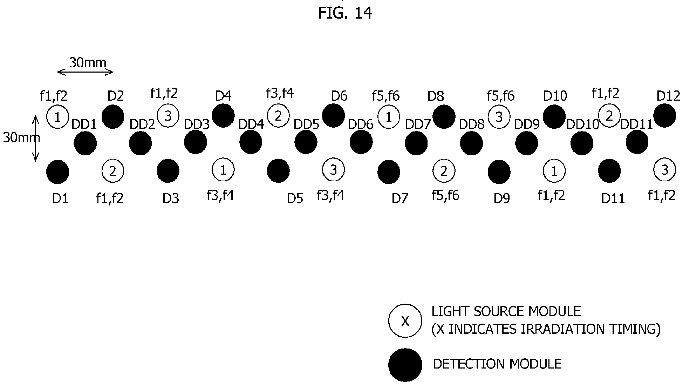

FIG. 14 is a diagram illustrating an example of a layout, corresponding to a multi-distance measurement, of the light source modules and the detection modules. The module layout in FIG. 14 is a module layout as viewed from outside when the head region attaching device is attached to the measurement examinee's region. In the example of FIG. 14, 13 modules as a total sum are disposed, i.e., totally 12 modules are disposed in the crosswise direction at the lattice points of the tetragonal lattice in two rows arranged in the vertical direction, and 11 modules are disposed at centers of squares of the tetragonal lattice. In the example of FIG. 14, the light source module 14 is disposed at the left upper edge of the lattice points of the tetragonal lattice, and the light source modules 14 and the detection modules 15 are alternately disposed in the crosswise direction. The light source modules 14 and the detection modules 15 are alternately disposed also in the vertical direction. The detection modules 15 are disposed at the centers of the squares of the tetragonal lattice. Accordingly, the module layout in FIG. 14 encompasses the 12 light source modules 14 and the 13 detection modules 15 (D1-D12, DD1-DD11). The numerals written inside of the light source module in FIG. 14 indicate the timings (time-division times) at which the rays of light are irradiated. Supposing that a division count of the time-division is "3", the irradiation timings are given in the sequence of 1, 2, 3 followed in loop by 1, and this sequence is repeated. The first period of time is notated by the time T1, and the second period of time is likewise notated by the time T2. The symbols [f1, f2] written in the periphery of the light source module indicate the frequencies at which the wavelengths λ1, λ2 (of the rays of light) to be irradiated are modulated. For example, in the light source module 14 at the left upper edge, the rays of light having the wavelength λ1 are modulated at the frequency f1 and then irradiated at the first period of time (T1); and the rays of light having the wavelength λ2 are modulated at the frequency f2 and then irradiated at T1. The detection module 15 of D1 detects the rays of light having the wavelength λ1, λ2 that are irradiated at the time T1 from the upper neighboring light source module 14 and modulated at the frequencies f1, f2, and detects the rays of light having the wavelength λ1, λ2 that are irradiated at the time T2 from the right neighboring light source module 14 and modulated at the frequencies f1, f2. The detection module 15 of DD1 also detects the rays of light having the wavelength λ1, λ2 that are irradiated at the time T1 from the left upper neighboring light source module 14 and modulated at the frequencies f1, f2, and detects the rays of light having the wavelength λ1, λ2 that are irradiated at the time T2 from the right lower neighboring light source module 14 and modulated at the frequencies f1, f2. A distance between the detection module 15 of DD1 and the neighboring the light source module 14 is about 21 mm shorter than the distance of 30 mm between the detection module 15 of D1 and the neighboring light source module 14. The layout such as this enables the signals of the plurality of distances to be acquired. This layout is configured to add only the detection module 15 as compared with the "2x12" layout of the tetragonal lattice, and therefore prevents other detection modules from being affected by optical noises and other equivalent factors. The time-division count and the modulation frequency count in this layout may be set the same as those in the "2x12" layout of the tetragonal lattice.

<Selection of Modulation Frequency>

When setting an intensity modulation frequency (actuation frequency) of the rays of light irradiated from the light source module 14, the respective light intensity modulation frequencies are selected under conditions given below in order to reduce the crosstalk. The actuation frequency is notated by fi (*i* is an arbitrary integer. For example, the actuation frequencies are notated by f1, f2, f3, f4, f5, f6), and, supposing that the AD converter performs an analog-digital conversion at a predetermined sample frequency fs, the sample frequency fs of the analog-digital conversion entails being an even-numbered multiple of each actuation frequency fi (Expression 1).
[Mathematical Expression 1]

$$fs = 2L \, x \, fi \ (L \ is \ natural \ number) \quad (Expression \ 1)$$

When there is a plurality of actuation frequencies, it may be sufficient to select such an even-numbered multiple of the frequency as to become a least common multiple thereof. This aims at sampling only a same equal number of positive values (alternatively, voltages higher than a reference voltage) and the same equal number of negative values (alternatively, voltages lower than the reference voltage) with respect to all the frequencies (other actuation frequencies) other than the actuation frequency of the predetermined signal in order to cancel the signals (undesired signals, signals related to other actuation frequencies) other than the predetermined signal on such an occasion that the multiplier multiplies the frequency of the predetermined signal when makinga lock-in detection. In the case of an odd-numbered multiple, it follows that the undesired signals do not cancel each other on a positive-negative basis, but are carried on the predetermined signals as noises (crosstalk) depending on an amplitude . The arithmetic unit calculates an average or a sum of the voltage values acquired a predetermined number of times *n*. Time (integrating time) (T = n/fs) for calculating the average or the sum entails being an integral multiple of each actuation signal cycle (1/fi) set by the arithmetic unit (Expression 2).
[Mathematical Expression 2]

$$T = \frac{1}{fs} = Mi \, x \, \frac{1}{fi} \quad (Mi \ is \ natural \ number) \quad (Expression \ 2)$$

Hereat, a beat (or hum) cycle Tb between the respective actuation frequencies is given by:
[Mathematical Expression 3]

$$Tb = \frac{1}{|fi - fj|} = \frac{1}{\left[\!\left[\dfrac{Mi}{T}\right]\!\right] - \left[\!\left[\dfrac{Mj}{T}\right]\!\right]} = \frac{T}{|Mi - Mj|} \quad (Expression \ 3)$$

A ratio between the integrating time (T=n/fs) and the beat cycle Tb is given by:
[Mathematical Expression 4]

$$\frac{T}{Tb} = \left| Mi - Mj \right| = N \quad (N \text{ is natural number}) \quad (Expression\ 4)$$

Thus, the integrating time becomes the integral multiple of the beat cycle, and hence a beat signal is cancelled by adding or averaging in the integrating time T. When Expression 4 is established, Expression 5 is established as below.
[Mathematical Expression 5]

$$N = \left| Mi - Mj \right|$$
$$= \left| fi\ x\ \frac{n}{fs} - fi\ x\ \frac{n}{fs} \right| = n \left| \frac{1}{Npi} - \frac{1}{Npj} \right| \quad (Expression\ 5)$$

(where, Npi is a sample count of the analog-digital conversion in the actuation signal cycle (1/fi)). When a pulse count of the actuation frequency fi contained in the integrating time T is given by ki (= n/Npi = fi x T), Expression 6 is established as follows:
[Mathematical Expression 6]

$$N = \left| ki - kj \right| \quad (Expression\ 6)$$

A difference between the mutual pulse counts becomes a natural number . Incidentally, when satisfying Expression 2, Expression 7 is established as follows:
[Mathematical Expression 7]

$$ki = fi\ x\ T = \left[ \frac{Mi}{T} \right] x\ T = Mi \quad (Expression\ 7)$$

Each frequency signal pulse count contained in the integrating time T is a natural number.
The conditions of at least Expressions 1 and 2 are satisfied simultaneously, and the frequency signals other than desired signals are thereby cancelled on the positive-negative basis through the averaging process and thus restrained, with the result that any crosstalk caused by the optical signals from the different light sources is not detected. Generally, there exists a plurality of combinations of the frequencies to satisfy the conditions given above, and it therefore follows that an optimal value is determined from a condition of a number of desired frequency types, a condition of desired sample frequencies and other equivalent conditions . When avoiding interferences with frequencies instanced by a commercial power frequency and an interior illumination frequency, the frequency is determined not to become integral multiples of these frequencies. The discussion made so far does not refer to phases of the respective actuation frequencies, and such a configuration is, however, available that an optimal phase is selected corresponding to a measurement condition and an environment by using phase conversion means and other equivalent means. Variations in phase have an effect of enabling variations in magnitude of apeak value of a total sum of quantities of detection rays of light from the plurality of light sources and variations in timing thereof.

(Operational Example)

<Setting of Frequency>

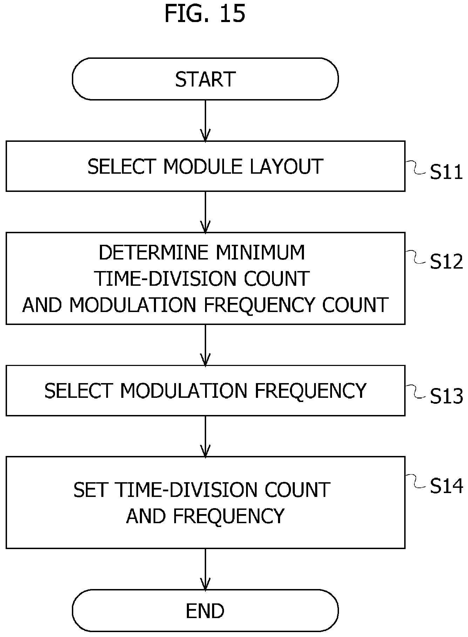

FIG. 15 is a flowchart illustrating an example of an operation flow of setting the frequency in the module. The head region attaching device 10 may also execute the processed to be executed by the user terminal 20.
In S11, the user, who is going to measure the variations in brain bloodflow of the head region, selects the layout of the modules (the layout of the light source modules and the detection modules) to be attached to the head region by using the user terminal 20. The selected module layout may be inputted by the user and other equivalent persons to the user terminal 20, and the module layout may also be selected by the user from within a plurality of patterns of the module layouts in a way that displays these patterns on the output unit 27 of the user terminal 20.
In S12, the user terminal 20 determines the minimum time-division count, based on the selected module layout. The user terminal 20 determines, based on the selected module layout, the minimum time-division count from the relationship to be stored in the storage means between the module layout, the minimum time-division count and other equivalent items as in FIG. 10.

In S12, the user terminal 20 determines the minimum time-division count and the modulation frequency on the basis of the selected module layout. The user terminal 20 determines, based on the selected module layout, the minimum time-division count and the minimum modulation frequency count from the relationship to be stored in the storage means between the module layout, the minimum time-division count and other equivalent items as in FIG. 10.

In S13, the user terminal 20 determines the modulation frequencies for the number of the minimum modulation frequencies determined in S12 as the modulation frequencies used in the light source module 14 and the detection module 15. As described above, the user terminal 20 selects the modulation frequencies, of which the modulation frequency count is determined in S12. The user terminal 20 selects, as described above, the sample frequency (sampling frequency), the integrating time and other equivalent items used in the light source module 14 and the detection module 15. The user terminal 20 allocates the modulation frequencies and the irradiation times used in the light source modules 14 and the detection modules 15 to the respective modules so that the rays of light modulated at the same frequency are not irradiated at the same timing within the predetermined range from the detection module 15.

In S14, the user terminal 20 sets the irradiation time, the modulation frequency, the sample frequency, the integrating time and other equivalent items in each light source module 14 and each detection module 15 of the head region attaching device 10. The irradiation time (timing), the modulation frequency and other equivalent items are thereby set in each of the light source modules 14 and the detection modules 15 of other module layouts, which are selected in S11.

(Others)

The apparatus is herein configured as the head region attaching device 10 attached to the head region, and the light source module 14 and the detection module 15 may, however, measure variations in bloodflow dynamics or the bloodflow in muscles by being pasted onto the skin (onto the muscles) other than the head region. The measurement of the variations in bloodflow dynamics and other equivalent elements in the muscles enables a calculation of oxygen metabolism in the muscles when taking exercise. Hereat, there are measured the variations in bloodflow in a position shallower from the skin than in the case of measuring the variations in brain bloodflow rate, and hence the interval between the light source module 14 and the detection module 15 is set smaller than 30 mm.

(Operation and Effect of Embodiment)

According to the configuration of the embodiment, the modulation frequency is properly selected, and the time-division measurements are combined, thereby making it possible to provide the technology of simultaneously preventing both the decrease in S/N ratio and the crosstalk and downsizing the apparatus by further reducing the types of the frequencies for use.

<<Non-Transitory Computer Readable Recording Medium>>

A program making a computer, other machines and apparatuses (which will hereinafter be referred to as the computer and other equivalent apparatuses) attain any one of the functions, may be recorded on a non-transitory recording medium readable by the computer and other equivalent apparatuses. The computer and other equivalent apparatuses are made to read and run the program on this non-transitory recording medium, whereby the function thereof may be provided.

Herein, the non-transitory recording medium readable by the computer and other equivalent apparatuses connotes a non-transitory recording medium capable of accumulating information instanced by data, programs and other equivalent information electrically, magnetically, optically, mechanically or by chemical action, which may be read from the computer and other equivalent apparatuses. The non-transitory recording medium is provided with computer components instanced by the CPU and the memory, in which the CPU may run the program.

Among these non-transitory recording mediums, the mediums removable from the computer and other equivalent apparatuses are exemplified by a flexible disc, a magneto-optic disc, a CD-ROM, a CD-R/W, a DVD, a DAT, an 8 mm tape, and a memory card.

A hard disc, a ROM (Read-Only Memory) and other equivalent recording mediums are given as the non-transitory recording mediums fixed within the computer and other equivalent apparatuses.

[Brief Description of Reference Numerals and Symbols]

10    head region attaching device
11    control unit
13    wireless communication unit
14    light source module
15    detection module
142   light source control unit
141   light source
151   detector
152   signal acquiring unit
153   AD converter
154   arithmetic unit
241   oscillation unit
242   frequency division unit
243   frequency division unit
244   signal selection unit
245   signal selection unit
246   light irradiation unit
251   light detection unit
252   amplifier
253   oscillation unit
254   frequency division unit
255   frequency division unit
256   signal selection unit
257   signal selection unit
258   multiplier
259   multiplier
260   AD converter
261   AD converter
262   arithmetic unit
263   communication unit
20    user terminal
21    CPU
22    memory
23    wireless communication unit
24    public circuit communication unit
25    display unit
26    operation unit
27    output unit
28    image capturing unit
29    positioning unit
2A    physical sensor unit

**Claims**

1. A biometric optical measurement apparatus comprising:

   one or a plurality of light source units to irradiate a measurement examinee's region with rays of light modulated at a predetermined modulation frequency;
   one or a plurality of light detection units to detect optical signals based on the rays of light irradiated from the light source units;
   a signal acquiring unit to multiply the optical signal detected by the light detection unit by a signal of the predetermined modulation frequency corresponding to each light source unit, and to acquire the multiplied signal as a signal irradiated from each light source unit;
   an analog-to-digital (AD) converter to AD-convert the signal acquired by the signal acquiring unit at a predetermined sample frequency; and
   an arithmetic unit to integrate the signals converted by the AD converter,
   the sample frequency being an even-numbered multiple of all the modulation frequencies,

time integrated by the arithmetic unit being an integral multiple of a modulation signal cycle as an inverse number of the modulation frequency.

2. The biometric optical measurement apparatus according to claim 1, wherein the modulation frequency is generated by frequency-dividing a predetermined frequency signal, and
the sample frequency of the AD conversion is determined by the predetermined frequency signal or by frequency-dividing the predetermined frequency signal.

3. The biometric optical measurement apparatus according to claim 1 or 2, wherein the light source unit irradiates the rays of light modulated at the predetermined modulation frequency at one of times divided by predetermined time-division.

4. The biometric optical measurement apparatus according to claim 3, further comprising a plurality of the light source units,
wherein of these light source units the light source units irradiating the rays of light at the same time and modulated at the same modulation frequency are so disposed as to be spaced at a predetermined or longer distance.

5. Abiometric optical measurement method by which a biometric optical measurement apparatus comprising: one or a plurality of light source units to irradiate a measurement examinee's region with rays of light modulated at a predetermined modulation frequency; and one or a plurality of light detection units to detect optical signals based on the rays of light irradiated from the light source units, executes:

multiplying the optical signal detected by the light detection unit by a signal of the predetermined modulation frequency corresponding to each light source unit, and acquiring the multiplied signal as a signal irradiated from each light source unit;
AD(analog-to-digital)-converting the acquired signal at a predetermined sample frequency; and
integrating the signals converted by the AD converter,
the sample frequency being an even-numbered multiple of all the modulation frequencies,
time to be integrated being an integral multiple of a modulation signal cycle as an inverse number of the modulation frequency.

FIG. 1

USER TERMINAL

20

N1

HEAD REGION
ATTACHING DEVICE

10

# FIG. 2

CONTROL UNIT — 11

13

WIRELESS COMMUNICATION UNIT

LIGHT SOURCE MODULE — 14

DETECTION MODULE — 15

HEAD REGION ATTACHING DEVICE

10

**FIG. 3**

| CPU | MEMORY |
|---|---|

21    22

WIRELESS COMMUNICATION UNIT   23
24

PUBLIC CIRCUIT
COMMUNICATION UNIT

DISPLAY UNIT   25

OPERATION UNIT   26

OUTPUT UNIT   27

IMAGE CAPTURING UNIT   28

POSITIONING UNIT   29

PHYSICAL SENSOR UNIT   2A

USER TERMINAL

20

FIG. 4

LIGHT SOURCE MODULE

LIGHT

| LIGHT SOURCE | | LIGHT SOURCE CONTROL UNIT |
|---|---|---|
| 142 | | 141 |

14

DETECTION MODULE

| DETECTOR | SIGNAL ACQUIRING UNIT | AD CONVERTER | ARITHMETIC UNIT |
|---|---|---|---|
| 151 | 152 | 153 | 154 |

15

MEASUREMENT EXAMINEE'S REGION

FIG. 5

LIGHT SOURCE MODULE

14

OSCILLATION UNIT
241

FREQUENCY DIVISION UNIT
242
. . .

SIGNAL SELECTION UNIT (λ1)
244

FREQUENCY DIVISION UNIT
243
. . .

SIGNAL SELECTION UNIT (λ2)
245

246

LIGHT IRRADIATION UNIT

LIGHT

MEASUREMENT EXAMINEE'S REGION

15

DETECTION MODULE

LIGHT DETECTION UNIT
253

AMPLIFIER
252

OSCILLATION UNIT
251

FREQUENCY DIVISION UNIT
254
. . .

SIGNAL SELECTION UNIT(λ1)
256

FREQUENCY DIVISION UNIT
255
. . .

SIGNAL SELECTION UNIT(λ2)
257

MULTIPLIER
258

MULTIPLIER
259

AD CONVERTER
260

AD CONVERTER
261

ARITHMETIC UNIT
262

COMMUNI-CATION UNIT
263

FIG. 6

30mm

LIGHT
SOURCE
MODULE

FIRST
DETECTION
MODULE

SECOND
DETECTION
MODULE

SCALP

14    LIGHT    15-1    15-2

HEAD
REGION

CEREBRAL CORTEX    LIGHT

| LIGHT SOURCE | WAVELENGTH | FREQUENCY |
|---|---|---|
| S1 | λ1 | f1 |
| S1 | λ2 | f2 |
| S2 | λ1 | f3 |
| S2 | λ2 | f4 |
| S3 | λ1 | f3 |
| S3 | λ2 | f4 |
| S4 | λ1 | f5 |
| S4 | λ2 | f6 |

RECTANGULAR WAVE SIGNAL
MODULATED AT FREQUENCY f1

TIME

T1 T2 T3 T4 T1 T2 T3 T4 T1 T2 T3 T4

FIG. 7

FIG. 8

LIGHT SOURCE MODULE
(X INDICATES IRRADIATION TIMING)

DETECTION MODULE

FIG. 9

LIGHT SOURCE MODULE
(X INDICATES IRRADIATION TIMING)

DETECTION MODULE

| MODULE LAYOUT (CROSSWISE × VERTICAL) | MINIMUM TIME-DIVISION COUNT | MINIMUM MODULATION FREQUENCY COUNT (2 WAVELENGTHS) |
|---|---|---|
| 1 × 2 | 1 | 2 |
| 1 × 3、1 × 4 | 2 | 2 |
| 1 × N （N ≧ 5） | 2 | 4 |
| 2 × N （N ＝ 2, 3） | N | 2 |
| 2 × N （N ≧ 4） | 3 | 4 |
| 3 × 3 | 3 | 4 |
| 3 × 4 | 4 | 4 |
| 3 × N （N ≧ 5） | 4 | 6 |
| M × N （M, N ≧ 4） | 4 | 6 |

FIG. 10

FIG. 11

| MODULE LAYOUT (CROSSWISE x VERTICAL) | MINIMUM TIME-DIVISION COUNT | MINIMUM MODULATION FREQUENCY COUNT (2 WAVELENGTHS) |
|---|---|---|
| 1 × 2 | 1 | 2 |
| 1 × N （N≧3） | 2 | 2 |
| 2 × 2 | 2 | 2 |
| 2 × N （N≧3） | 3 | 2 |
| 3 × 3 | 3 | 2 |
| M × N （M≧3，N≧4） | 4 | 2 |

FIRST DIRECTION

N-NUMBER OF MODULES

M-NUMBER OF MODULES

SECOND DIRECTION

FIG. 12

○ LIGHT SOURCE MODULE

● DETECTION MODULE

FIG. 13

| MODULE LAYOUT (FIRST DIRECTION × SECOND DIRECTION) | MINIMUM TIME-DIVISION COUNT | MINIMUM MODULATION FREQUENCY COUNT (2 WAVELENGTHS) |
|---|---|---|
| 2 × N (N = 2, 3) | N | 2 |
| 2 × N (N ≧ 4) | 3 | 4 |
| 3 × 3 | 3 | 4 |
| M × N (M ≧ 3, N ≧ 4) | 4 | 4 |

FIG. 14

30mm

30mm

f1,f2    D2    f1,f2    D4    f3,f4    D6    f5,f6    D8    f5,f6    D10    f1,f2    D12

① DD1   DD2 ③ DD3   DD4 ② DD5   DD6 ① DD7   DD8 ③ DD9   DD10 ② DD11

D1    ②    D3    ①    D5    ③    D7    ②    D9    ①    D11    ③

    f1,f2    f3,f4    f3,f4    f5,f6    f1,f2    f1,f2

Ⓧ   LIGHT SOURCE MODULE
      (X INDICATES IRRADIATION TIMING)

●   DETECTION MODULE

# FIG. 15

START

SELECT MODULE LAYOUT — S11

DETERMINE MINIMUM
TIME-DIVISION COUNT
AND MODULATION FREQUENCY COUNT — S12

SELECT MODULATION FREQUENCY — S13

SET TIME-DIVISION COUNT
AND FREQUENCY — S14

END

## INTERNATIONAL SEARCH REPORT

| International application No. |
| --- |
| PCT/JP2017/005003 |

A. CLASSIFICATION OF SUBJECT MATTER

*A61B10/00*(2006.01)i

According to International Patent Classification (IPC) or to both national classification and IPC

B. FIELDS SEARCHED

Minimum documentation searched (classification system followed by classification symbols)

A61B10/00

Documentation searched other than minimum documentation to the extent that such documents are included in the fields searched

| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 2000-300569 A (Hitachi Medical Corp.), 31 October 2000 (31.10.2000), fig. 1, 3 (Family: none) | 1-5 |
| Y | JP 2012-161375 A (University of Tsukuba), 30 August 2012 (30.08.2012), fig. 1, 2 (Family: none) | 1-5 |
| A | JP 2015-528334 A (ViOptix, Inc.), 28 September 2015 (28.09.2015), & US 2014-46152 A1 & EP 2844145 A1 & CN 104411241 A | 1-5 |

☒ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>09 March 2017 (09.03.17) | Date of mailing of the international search report<br>21 March 2017 (21.03.17) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

| International application No. |
| --- |
| PCT/JP2017/005003 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2006-187641 A (Hitachi, Ltd.), 20 July 2006 (20.07.2006), (Family: none) | 1-5 |
| A | JP 2008-307399 A (Hitachi, Ltd.), 25 December 2008 (25.12.2008), (Family: none) | 1-5 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H9149903 B
- JP 2008178563 A

**Non-patent literature cited in the description**

- **MCCORMICK PW ; STEWART M ; LEWIS G ; DU-JOVNY M ; AUSMAN JI.** Intracerebral penetration of infrared light. *J Neurosurg,* 1992, vol. 76 (2), 315-8
- **ZEFF BW ; WHITE BR ; DEHGHANI H ; SCHLAG-GAR BL ; CULVER JP.** Retinotopic mapping of adult human visual cortex with high-density diffuse optical tomography. *Proc Natl Acad Sci U S A.,* 2007, vol. 104, 12169-12174